(19) Europäisches Patentamt
European Patent Office
Office européen des brevets

(11) Numéro de publication : **0 373 050 B1**

# (12) FASCICULE DE BREVET EUROPEEN

(45) Date de publication du fascicule du brevet : **28.04.93 Bulletin 93/17**

(51) Int. Cl.[5] : **C07C 45/00,** C07C 49/00

(21) Numéro de dépôt : **89403335.6**

(22) Date de dépôt : **01.12.89**

## (54) Perfectionnement à la préparation de cétones par l'acylation de composés organo-manganeux.

(30) Priorité : **09.12.88 FR 8816195**

(43) Date de publication de la demande : **13.06.90 Bulletin 90/24**

(45) Mention de la délivrance du brevet : **28.04.93 Bulletin 93/17**

(84) Etats contractants désignés : **AT BE CH DE ES GB GR IT LI LU NL SE**

(56) Documents cités :
**TETRAHEDRON LETTERS, no. 36, 1976, pages 3155-3156, Pergamon Press; G. CAHIEZ et al.: "Réactivité des dérivés organo-manganeux. I-action sur les chlorures d'acides"**
**SYNTHESIS, janvier 1984, pages 37-40; G. FRIOUR et al.: "Organomanganous Reagents; IX. Preparation of various halogenated, alkoxylated, aryloxylated, and arylsulfenylated ketones from correspondingly functionalized carboxylic acid chlorides or anhydrides"**

(56) Documents cités :
**JACS, vol. 71, decembre 1949, pages 4141-4142; N.C. COOK et al.: "Grignard reactions. XX. Effect of cuprous chloride on the reaction of t-Butylmagnesium chloride and trimethylacetyl chloride"**
**JACS, vol. 66, mars 1944, pages 368-371; M.S. KHARASCH et al.: "Factors determining the course and mechanisms of Grignard reactions. XIII. The effect of metallic halides on the reaction of sterically hindered acid halides with methylmagnesium iodide"**

(73) Titulaire : **SOCIETE NATIONALE ELF AQUITAINE**
**Tour Elf, 2, Place de la Coupole, La Défense 6**
**F-92400 Courbevoie (FR)**

(72) Inventeur : **Cahiez, Gérard**
**68 Rue de Turbigo**
**F-75003 Paris (FR)**
Inventeur : **Laboue, Blandine, Service de Monsieur Cahiez**
**Université P. et M. Curie, Tour 44-45 2ème étage**
**4 Place Jussieu, F-75252 Paris Cédex 05 (FR)**
Inventeur : **Tozzolino, Pierre**
**Chemin Carrerot Serres-Morlaas**
**F-64160 Morlaas (FR)**

(74) Mandataire : **Bertrand, Didier et al**
**Cabinet FEDIT-LORIOT 38, Avenue Hoche**
**F-75008 Paris (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



Jouve, 18, rue Saint-Denis, 75001 PARIS

## Description

L'invention se rapporte à un perfectionnement du procédé connu, qui consiste à produire une cétone, par l'action d'un agent d'acylation au sein d'un solvant organique sur un organo-manganeux, en particulier sur un organo-manganeux mixte.

Ce procédé connu a apporté un progrès important à la technique concernée, et a permis l'obtention d'un grand nombre de cétones très diverses, pouvant porter différents groupes fonctionnels ou non. L'utilisation d'organo-manganeux mixtes a rendu possible le travail dans des conditions plus douces, industriellement plus pratiques que celles de la technique antérieure. Des renseignements utiles sur ce sujet se trouvent dans l'article de G. CAHIEZ, "Les Organo-manganeux : utilisations en synthèse organique", paru dans "L'Actualité Chimique" de Septembre 1984. On y trouve, entre autres, un tableau de 24 types de cétones, qui montre la diversité des produits qu'il est possible d'obtenir par le procédé en question, avec de bons rendements.

Il est notamment connu que les composés organo-manganeux iodés sont facilement acylés par un chlorure d'acide dans l'éther. Cette réaction permet de préparer avec de bons rendements de nombreuses cétones. Cependant, la préparation de cétones encombrées à partir d'un organo-manganeux iodé possédant un groupe alkyle tertiaire ou secondaire nécéssite des précautions particulières en raison de l'instabilité de ces composés : excès importants d'organo-manganeux, complexation de cet organo-manganeux par le tétrahydrofuranne, abaissement de la température jusqu'à -4O°C. Malgrè toutes ces précautions, les cétones encombrées, correspondantes, sont obtenues avec des rendements relativement moyens. D'ailleurs,quels que soient les résultats obtenus, des considérations économiques interdisent, d'une façon générale, l'utilisation d'organo-manganeux iodés à l'échelle industrielle.

Il est également connu que la plupart des organo-manganeux chlorés (notamment ceux qui ne possèdent pas de groupe alkyle secondaire ou tertiaire) sont acylés par des anhydrides symétriques dans le tétrahydrofuranne avec des rendements convenables, bien que légèrement inférieurs à ceux que l'on obtient avec ces organo-manganeux iodés dans l'éther. Néanmoins, l'emploi d'un anhydride symétrique présente l'inconvénient d'utiliser un seul des deux restes acyle mis en jeu.

En revanche l'utilisation d'autres agents acylants, tels que les anhydrides mixtes (R'COOCOOR") et les chlorures d'acide a longtemps posé problème ("Activité des organo-manganeux", TETRAHEDRON, vol. 4O, N° 4 page 683 à 693, 1984). En effet, lors de la réaction d'un anhydride mixte avec un organo-manganeux chloré, dans le tétrahydrofuranne, on constate qu'il se forme en majeure partie de l'ester à coté d'un peu de cétone. En ce qui concerne les chlorures d'acide, après certains échecs dus à l'ouverture du cycle du tétrahydrofuranne en présence de sels de manganèse, des précautions opératoires ont permis de les utiliser dans un grand nombre de cas ; néanmoins, la préparation de certaines cétones s'est révélée impossible dans des conditions de rendement acceptables. En particulier, la réaction d'un chlorure d'acide, dans le tétrahydrofuranne avec des organo-manganeux $RM_nCl$, dans lesquels R est un groupe méthyle, alkyle secondaire ou tertiaire, ou aryle conduit à des rendements médiocres ou parfois nuls.

L'amélioration, apportée par la présente invention , permet de remédier à ces inconvénients avec de bons rendements en cétone,quel que soit l'agent d'acylation utilisé et quelle que soit la structure de l'organo-manganeux.

Le procédé selon l'invention, qui consiste à produire une cétone par l'action d'un agent d'acylation sur un composé organo-manganeux au sein d'un solvant organique d'après la réaction

$$(1) \quad \ldots \quad RMnX + R'COZ \xrightarrow[\text{organique}]{\text{solvant}} R\text{-}CO\text{-}R' + MnXZ \;,$$

est caractérisé en ce que l'on introduit dans le milieu un composé du cuivre monovalent ou divalent. Il est préférable d'opérer cette introduction dès le début de la réaction.

Parmi les composés du cuivre, utilisables selon l'invention, il va de soi qu'il y a intérêt à employer ceux qui sont les plus accessibles dans la pratique, notamment les chlorures,bromures, acétates, propionates,stéarates ou d'autres sels d'acides aliphatiques.

Bien que la proportion de catalyseurs cuivreux ou cuivriques puissent varier largement, elle est en général de 1 à 1O atomes de cuivre pour 1OO moles de composé organo-manganeux et de préference 2 à 6 atomes.

Les effets bénéfiques de l'addition de catalyseur cuivreux ou cuivrique se font sentir avec de nombreux composés organo-manganeux et avec divers agents d'acylation, si l'acylation est effectuée à des températures entre -50°C et l'ambiante, avec addition de catalyseur,comme indiqué ci-dessus.

Les agents acylants R'COZ selon l'invention sont tels qu'ils peuvent être des halogénures d'acides R'COX (X halogène), des anhydrides $(R'CO)_2O$, R'COOCOR", R'COOCOOR". R' et R", semblables ou différents peuvent être des groupes organiques très variés, notamment aliphatiques, aryliques, cycloaliphatiques, etc., pouvant porter des groupes fonctionnels. Les améliorations, apportées par l'invention, sont particulièrement nettes dans le cas des chlorures d'acides et des anhydrides mixtes.

Les organo-manganeux RMnX selon l'invention peuvent être des organo-manganeux mixtes, X étant un halogène, R un groupe organique, notamment aliphatique, arylique et cycloaliphatique pouvant, le cas échéant, porter des groupes fonctionnels. Les améliorations apportées sont particulièrement nettes dans le cas des chlorures d'acides lorsque R est un groupe méthyle, alkyle secondaire ou tertiaire, ou aryle. Le perfectionnement, selon l'invention, s'applique particulièrement bien aux préparations de cétones en partant des organo-manganeux mixtes.

L'amélioration due à l'adjonction d'un composé du Cu est d'autant plus surprenante que, pour des réactions analogues avec des organo-magnésiens, on n'a trouvé aucun effet catalytique avec des sels de Cu (brevet US 4298542, Tableau 1, col. 8-9).

Les différents paramètres du procédé sont les mêmes que dans la technique antérieure. Ainsi, en ce qui concerne le solvant, il est de préférence constitué par un éther, en particulier un éther cyclique tel que le tétrahydrofuranne. Des mélanges d'éthers avec d'autres solvants peuvent être utilisés, par exemple avec l'acétate d'éthyle, le carbonate d'éthyle, l'acétonitrile, ou des hydrocarbures aliphatiques ou aromatiques, par exemple benzène, toluène, hexane, cyclohexane, etc. Les concentrations des composés,réagissant dans le solvant, peuvent varier, mais il est pratique de travailler avec des solutions ou/et suspensions contenant 0,1 à 2 mole de chacun de ces composés par litre de solvant, ou mieux 0,3 à 0,7 mole/litre.

Les exemples, donnés plus loin, montrent les gains en rendement obtenus dans différents cas d'application du procédé selon l'invention. Ils ont été mis en oeuvre dans les conditions suivantes, lorsque l'organo-manganeux est RMnCl.

A une suspension de 52 mmoles de RMnCl dans 100 ml de THF on ajoutait 2,5 mmoles de CuCl puis 50 mmoles d'un chlorure d'acide R'COCl, sous agitation à l'abri de l'air. Cette opération avait lieu entre 0°C et -50°C. On laissait ensuite le milieu réactionnel se réchauffer à la température ambiante, et on l'agitait pendant 2 heures. Après ce temps, le milieu était refroidi à -10°C et additionné de 60 ml d'une solution aqueuse de HCl 1N, pour éliminer les sels de Mn. La phase aqueuse était décantée et les matières organiques y contenues étaient extraites par 2 fois 50 ml d'éther. Les phases organiques réunies étaient ensuite lavées avec 50 ml d'une solution aqueuse saturée de $NaHCO_3$. Après séchage sur $MgSO_4$ et évaporation des solvants sous vide, la cétone formée était séparée par distillation.

Lorsque le chlorure d'acide était remplacé par un anhydride mixte ( $RCOOCOOC_2H_5$), le mode opératoire était le même.

EXEMPLES 1 à 8

Dans cette série d'essais, on faisait réagir le chlorure de l'acide octanoïque $C_{17}H_{15}$-COCl avec différents chlorures organo-manganeux mixtes RMnCl, dans du THF, dont la température au moment du mélange est indiquée au Tableau ci-après. Dans chaque cas on effectuait, selon le mode opératoire décrit plus haut, un essai sans catalyseur et un avec 5 atomes de $Cu^+$, sous la forme de CuCl, pour 100 moles de chlorure d'acide utilisé. Le Tableau donne, comme résultats, les rendements en la cétone R-CO-$C_7H_{15}$ obtenue dans chaque cas par la réaction :

$$RMnCl + C_7H_{15}COCl \rightarrow RCOC_7H_{15} + MnCl_2$$

TABLEAU I

| Ex. | R | Température | CuCl | Rendement % |
|---|---|---|---|---|
| 1 | 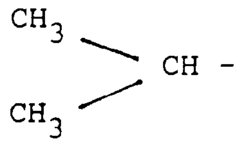 | -50°C | 0 | 69 |
| 2 | " | " | 5% | 93 |
| 3 | 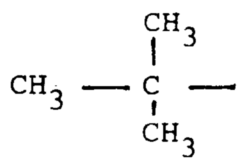 | " | 0 | 0 |
| 4 | " | " | 5% | 92 |
| 5 | 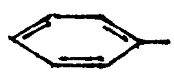 | -20°C | 0 | 55 |
| 6 | " | " | 5% | 63 |
| 7 | $CH_3$ | " | 0 | 40 |
| 8 | " | " | 5% | 91 |

On peut constater une forte amélioration du rendement dans les préparations faites en présence de CuCl. Elle est frappante dans le cas où R est tert.butyle (exemples 3 et 4), dont l'effet d'empêchement stérique n'est pas observé en présence de cuivre.

EXEMPLES 9 et 10

Des préparations analogues à celles des exemples précédents sont effectuées avec un chlorure d'acide moins réactif , celui de l'acide benzoïque, $C_6H_5$-COCl, que l'on mélange, dans du THF à O°C, avec du chlorure de tert. butyl manganèse ; sans catalyseur , le rendement en cétone tBuCOPh est pratiquement nul ;avec 5 moles de CuCl pour 100 moles de $C_6H_5COCl$ il ressort à 77%, donc effet remarquable.

EXEMPLE 11

Essai comparatif pour les exemples 1 et 2.

Jusqu'à présent, par la technique connue, les meilleurs résultats de préparation de l'isopropyl heptyl cétone étaient obtenus par l'action au sein de l'éther d'une mole de chlorure de l'acide octanoïque $C_6H_{15}COCl$ sur 1,5 mole d'iodure d'isopropyl manganèse, $(CH_3)_2CH$-Mn-I complexé dans 5 équivalents de THF,le mélange étant fait entre -30° et -40°C. On avait alors un rendement de 71% contre les 69% de l'exemple 1, obtenu avec seulement 1 mole de $(CH_3)_2CH$-Mn-Cl. Les 93% atteints dans l'exemple 2, grâce à l'addition de CuCl, présentent un grand intérêt non seulement par l'augmentation du rendement de 22%, mais aussi du fait de l'emploi d'un organomanganeux mixte, chloré, moins cher et plus stable que le dérivé iodé correspondant.

EXEMPLES 12 à 16

Dans cette série d'essais on faisait réagir différents anhydrides mixtes R'-COO-$COOC_2H_5$ avec différents organomanganeux RMnCl dans du THF dont la température au moment du mélange est comprise entre -10°C et 20°C en présence de 3% molaire (par rapport à l'anhydride) de CuCl. Le Tableau II donne comme résultats les rendements obtenus en cétone R-CO-R'.

| Ex. | R | R' | Rendement % |
|---|---|---|---|
| 12 | Butyl | Heptyl | 81 |
| 13 | Butyl | $(CH_3)_2$-C=CH | 76 |
| 14 | Butyl | Phényl | 60 |
| 15 | t-Butyl | Heptyl | 76 |
| 16 | Phényl | Heptyl | 78 |

EXEMPLE 17

Dans des conditions similaires à celles de l'exemple 12, on a fait réagir, en l'absence de CuCl, $C_7H_{15}$-COO-$COOC_2H_5$ avec $C_4H_9$MnCl ; le rendement en cétone était de l'ordre de 30% seulement.

En outre la réaction est peu reproductible. On constate donc que l'addition de CuCl à l'exemple 12 permet d'améliorer notablement le rendement.

**Revendications**

1. Procédé de préparation de cétones par la réaction entre un agent d'acylation et un composé organo-manganeux, au sein d'un solvant organique, caractérisé en ce que la réaction est catalysée par l'adjonction d'un composé cuivreux ou cuivrique.

2. Procédé suivant la revendication 1, caractérisé en ce que la quantité de catalyseur est telle qu'il y ait 1 à 10 atomes de Cu, et de préférence 2 à 6 atomes, pour 100 moles de composé organo-manganeux.

3. Procédé suivant la revendication 1 ou 2, caractérisé en ce que le composé cuivreux ou cuivrique est le chlorure, bromure, acétate ou un autre sel d'acide aliphatique.

4. Procédé suivant une des revendications précédentes, dans lequel l'agent d'acylation est un halogénure d'acide ou un anhydride,de préférence un chlorure d'acide ou un anhydride mixte.

5. Procédé suivant une des revendications précédentes, dans lequel le composé organo-manganeux est du type RMnX, $R_2$Mn ou $R_3$MnLi, et plus particulièrement RMnCl, R étant tout groupe organique pouvant porter des substituants ou des groupes fonctionnels, et X étant un halogène.

6. Procédé suivant la revendication 5, dans lequel R est méthyle, alkyle secondaire ou tertiaire, ou aryle.

7. Procédé suivant une des revendications précédentes, dans lequel le solvant est un éther, en particulier un éther cyclique tel que le tétrahydrofuranne.

8. Procédé suivant la revendication 7, dans lequel l'éther est en mélange avec un autre solvant, notamment l'acétate d'éthyle, le carbonate d'éthyle, l'acétonitrile ou un hydrocarbure aliphatique ou aromatique.

9. Procédé suivant une des revendications 1 à 8, dans lequel l'agent d'acylation et le composé organo-manganeux se trouvent, chacun, à la concentration de 0,1 à 2 moles, et de préférence 0,3 à 0,7 mole, par litre de solvant.

10. Procédé suivant une des revendications précédentes, caractérisé en ce que les dissolution ou/et dispersion des réactifs pour leur mise en contact a lieu à une température entre 0°C et -50°C, après quoi on laisse revenir le milieu réactionnel à la température ambiante.

## Patentansprüche

1. Verfahren zur Herstellung von Ketonen durch Umsetzung eines Acylierungsmittels und einer Organo-Manganverbindung in einem organischen Lösungsmittel, dadurch gekennzeichnet, daß die Umsetzung durch Zugabe einer Kupfer(I)- oder Kupfer(II)verbindung katalysiert wird.

2. Verfahren nach Anspruch 1, dadurch gekennzeichnet, daß die Menge des Katalysators so ist, daß er 1 bis 10 Cu-Atome, vorzugsweise 2 bis 6 Atome, auf 100 mol Organo-Manganverbindung aufweist.

3. Verfahren nach Anspruch 1 oder 2, dadurch gekennzeichnet, daß die Kupfer(I)- oder Kupfer(II)verbindung Chlorid, Bromid, Acetat oder ein anderes Salz einer aliphatischen Säure ist.

4. Verfahren nach einem der vorausgegangenen Ansprüche, bei dem das Acylierungsmittel ein Säurehalogenid oder ein Anhydrid, vorzugsweise ein Säurechlorid oder gemischtes Anhydrid, ist.

5. Verfahren nach einem der vorausgegangenen Ansprüche, bei dem die Organo-Manganverbindung dem Typ RMnX, $R_2Mn$ oder $R_3MnLi$, insbesondere RMnCl, angehört, wobei jede R organische Gruppe ist, die Substituenten oder funktionelle Gruppen tragen kann und X für ein Halogenid steht.

6. Verfahren nach Anspruch 5, bei dem R Methyl, sekundäres oder tertiäres Alkyl oder Aryl bedeutet.

7. Verfahren nach einem der vorausgegangenen Ansprüche, bei dem das Lösungsmittel ein Ether, insbesondere ein cyclischer Ether wie Tetrahydrofuran, ist.

8. Verfahren nach Anspruch 7, bei dem der Ether ein Gemisch mit einem weiteren Lösungsmittel, insbesondere Ethylacetat, Ethylcarbonat, Acetonitril oder mit einem aliphatischen oder aromatischen Kohlenwasserstoff, ist.

9. Verfahren nach einem der Ansprüche 1 bis 8, bei dem das Acylierungsmittel und die Organo-Manganverbindung jeweils eine Konzentration von 0,1 bis 2 mol, vorzugsweise 0,3 bis 0,7 mol, pro Liter Lösungsmittel aufweisen.

10. Verfahren nach einem der vorausgegangenen Ansprüche, dadurch gekennzeichnet, daß die Auflösung und/oder Dispersion der Reaktionspartner zu ihrer Vermischung bei einer Temperatur zwischen 0 °C und -50 °C erfolgt, woraufhin man das Reaktionsgemisch auf Umgebungstemperatur zurückkehren läßt.

## Claims

1. Process of preparation of ketones by the reaction between an acylation agent and an organomanganous comnound in an organic solvent, characterised in that the reaction is catalysed by the addition of a cuprous or cupric compound.

2. Process according to claim 1, characterised in that the quantity of the catalyst is such that there is 1 to 10 atoms of Cu and preferably 2 to 6 atoms per 100 moles of organo-manganous compound.

3. Process according to claim 1 or 2, characterised in that the cuprous or cupric compound is the chloride, bromide, acetate or other aliphatic acid salt.

4. Process according to any of the preceding claims, in which the acylation agent is an acid halide or an anhydride, preferably an acid chloride or a mixed anhydride.

5. Process according to any of the preceding claims, in which the organo-manganous compound is of the type RMnX, $R_2Mn$, or $R_3MnLi$, and more particularly, RMnCl, R being any organic group which can carry substituents or functional groups, X being a halogen.

6. Process according to claim 5, in which R is methyl, secondary or tertiary alkyl ou aryl.

7. Process according to any of the preceding claims, in which the solvent is an ether, in particular a cyclic ether such as tetrahydrofuran.

8. Process according to claim 7, in which the ether is in admixture with another solvent, particularly ethyl acetate, ethyl carbonate in acetonitrile or an aliphatic or aromatic hydrocarbon.

9. Process according to any of claims 1 to 8 in which the acylation agent and the organomanganous compound are each present in a concentration from 0.1 to 2 mole and preferably 0.3 to 0.7 mole per litre of solvent.

10. Process according to any of the preceding claims, characterised in that dissolution and/or dispersion of the reactants to put them in contact takes place at a temperature between 0°C and -50°C, the reaction medium being thereafter left back to ambient temperature.